# EUROPEAN PATENT APPLICATION

(11) **EP 3 009 098 A1**
(43) Date of publication of application: **20.04.2016**
(21) Application number: 14810376.5
(22) Date of filing: 10.06.2014
(51) Int. Cl.: A61B 90/00, G02B 21/22

(54) **MICROSCOPE SYSTEM FOR SURGERY**

(30) Priority: 10.06.2013 JP 2013121746
(71) Applicant: Sumitomo Electric Industries, Ltd., Osaka-shi, Osaka 541-0041 (JP); Kyoto University, Kyoto-shi, Kyoto 606-8501 (JP)
(72) Inventor: OKUNO, Takuya, Yokohama-shi Kanagawa 244-8588 (JP); SOGAWA, Ichiro, Yokohama-shi Kanagawa 244-8588 (JP); SUGANUMA, Hiroshi, Yokohama-shi Kanagawa 244-8588 (JP); ISHII, Akira, Kyoto-shi Kyoto 606-8501 (JP); CHIHARA, Hideo, Kyoto-shi Kyoto 606-8501 (JP)
(74) Representative: Hoffmann Eitle
(86) International application number: PCT/JP2014/065349
(87) International publication number: WO 2014/199984

(57) **Abstract**

The position of a target substance existing on the inside of a tissue is grasped in a simpler method. From a first image indicating an intensity distribution of radiation from a subject 5 in a first wavelength region and a second image indicating an intensity distribution of radiation from the subject in a second wavelength region, a surgical microscope system 1 obtains image data of a third image indicating a position of a target substance. Output data obtained by superposing the image data of the third image onto shape image data further includes information indicating the target substance in addition to the image indicating the surface shape of the subject 5. Therefore, the position of the target substance existing on the inside of a tissue can be grasped non-invasively by referring to the output data. Further, the surgical microscope system 1 can grasp the position of the target substance on the inside of the tissue by capturing the first and second images separately from the shape image data and thus makes it possible to grasp the position of the target substance in a method simpler than conventional methods.

## Description

### Technical Field

The present invention relates to a microscope system for surgery (surgical microscope system).

### Background Art

As a method for non-destructively observing the inside of a tissue in a typical surgical microscope system, a method which accumulates a fluorescent dye in an object to be observed and observes the fluorescence of the fluorescent dye has been under study as in the invention described in Patent Literature 1, for example.

### Citation List

### Patent Literature

Patent Literature 1: Japanese Translated International Application Laid-Open No. 2009-525495

### Summary of Invention

### Technical Problem

When performing a bypass operation and the like with respect to diseases resulting from arteriosclerosis caused by fatty plaques adhering to inner walls of blood vessels and the like, for example, the method described in Patent Literature 1 can visualize bloodstreams, but fluorescent dyes which specifically bind to lipids are hard to obtain in general, thus making it difficult to visualize the fatty plaques themselves. It is also necessary for the method described in Patent Literature 1 to administer the fluorescent dye to a patient beforehand, so as to accumulate it in the object, which increases the burden on the patient.

In view of the foregoing, it is an object of the present invention to provide a surgical microscope system which can grasp in a simpler method the position of a target substance existing on the inside of a tissue.

### Solution to Problem

For achieving the above-mentioned object, the surgical microscope system in accordance with an embodiment of the present invention comprises a near-infrared light source for emitting illumination light at capture wavelengths including at least two wavelength regions within a wavelength range of 800 nm to 2500 nm; first imaging means, having a light detection band at the capture wavelengths, the first imaging means configured to capture an image indicating an intensity distribution of radiation from a subject irradiated with the illumination light from the near-infrared light source and to output image data; second imaging means configured to capture an image indicating a surface shape of the subject at a position provided with the first imaging means and to output shape image data; arithmetic means configured to produce output data indicating a position of a target substance included in the subject according to the image data output from the first imaging means and the shape image data output from the second imaging means; and display means configured to display the output data produced by the arithmetic means; wherein the capture wavelengths include a first wavelength region and second wavelength region, the first wavelength region having a width of 50 nm or less containing at least wavelengths within wavelength ranges of 1185 to 1250 nm and 1700 to 1770 nm and the second wavelength region being different from the first wavelength region; wherein the first imaging means configured to capture a first image and second image, the first image indicating the intensity distribution of the radiation from the subject in the first wavelength region and the second image indicating the intensity distribution of the radiation from the subject in the second wavelength region and to output respective image data; wherein the arithmetic means configured to generate image data of a third image indicating the position of the target substance according to the image data of the first image and the image data of the second image and to produce the output data by superposing the image data of the third image onto the shape image data output from the second imaging means.

The above-mentioned surgical microscope system obtains the image data of the third image indicating the position of a target substance from the first image indicating the intensity distribution of radiation from the subject in the first wavelength region and the second image indicating the intensity distribution of radiation from the subject in the second wavelength region. The output data obtained by superposing the image data of the third image onto the shape image data further includes information indicating the target substance in addition to the image indicating the surface shape of the subject. Therefore, the position of the target substance existing on the inside of a tissue can be grasped non-invasively. Further, the above-mentioned surgical microscope system can grasp the position of the target substance on the inside of the tissue by capturing the first and second images separately from the shape image data and thus makes it possible to grasp the position of the target substance in a method simpler than conventional methods.

Here, the second wavelength region may be included in a wavelength range of 1235 to 1300 nm and/or 1600 to 1680 nm.

Selecting a wavelength within the above-mentioned wavelength range as the second wavelength region can further enhance the accuracy in the position of the target substance specified by the third image obtained from the first and second images.

Here, the arithmetic means is configured to generate the image data of the third image by calculating a ratio between the intensity of the radiation in the image data of the first image and the intensity of the radiation in the image data of the second image.

Generating the image data of the third image by using the ratio between the intensity of the radiation in the image data of the first image and the intensity of the radiation in the image data of the second image as mentioned above yields the third image free of the unevenness in image caused by differences in intensity of light among pixels and the like.

The display means may display the output data after adjusting the luminance therein according to data in a part of the region indicated to have the target substance in the third image.

This enables luminance adjustment conforming to the luminance in the surroundings of the target substance by performing the luminance adjustment in the output data according to the data in the region indicated to have the target substance.

An optical filter selectively transmitting therethrough light having any of the capture wavelengths including the at least two wavelength regions may be provided on an optical path from the near-infrared light source to the first imaging means.

Here, the system may be configured such that the optical filter is configured to alternately selectively transmit therethrough light in the first wavelength region and light in the second wavelength region, the first imaging means is configured to capture the first and second images during when the optical filter transmits therethrough the light in the first wavelength region and the light in the second wavelength region, respectively, so as to capture the first and second images alternately and output image data, and the arithmetic means is configured to generate the image data of the third image according to image data acquired as outputs from the first imaging means and image data acquired most recently before acquiring the former image data.

Generating the image data of the third image according to the image data acquired as outputted from the first imaging means and image data acquired most recently before acquiring the former image data as mentioned above can continuously yield image data of the third image, whereby the output data can be displayed closer to real time.

The system may be configured such that the optical filter has a is configured to have first time zone for selectively transmitting therethrough light in the first region and a second time zone for selectively transmitting therethrough light in the second region, the first imaging means is configured to capture a plurality of first images in the first time zone and a plurality of second images in the second time zone, and the arithmetic means is configured to generate the image data of the third image according to data obtained by integrating image data of the plurality of first images captured in the first time zone and data obtained by integrating image data of the plurality of second images captured in the second time zone.

In this case, image data of the third image is generated according to the data integrating image data of a plurality of first images captured in the first time zone and the data integrating image data of a plurality of second images captured in the second time zone. Thus using the integrated data improves the SN ratio in the image data, thereby making it possible to grasp the position of the target substrate with a higher accuracy.

The optical filter may be arranged on an optical path from the subject to the first imaging means.

The near-infrared light source may include a first light source for emitting light in the first wavelength region and a second light source for emitting light in the second wavelength region.

The system may thus be configured to include the first and second light sources and acquire two images by switching between the light sources.

Here, the system may be configured such that the near-infrared light source is configured to emit the light in the first wavelength region and the light in the second wavelength region alternately, the first imaging means is configured to capture the first image during a time when the light in the first wavelength region is emitted and the second images during a time when the light in the second wavelength region is emitted so as to capture the first and second images alternately and output image data, and the arithmetic means is configured to generate the image data of the third image according to image data acquired as outputted from the first imaging means and image data acquired most recently before acquiring the former image data.

The system may be configured such that the near-infrared light source is configured to have a first time zone for selectively emitting the light in the first region and a second time zone for selectively emitting the light in the second region, the first imaging means is configured to capture a plurality of first images in the first time zone and a plurality of second images in the second time zone, and the arithmetic means is configured to generate the image data of the third image according to data obtained by integrating image data of the plurality of first images captured in the first time zone and data obtained by integrating image data of the plurality of second images captured in the second time zone.

### Advantageous Effects of Invention

The present invention can provide a surgical microscope system which makes it possible to observe in a simpler method the position of a target substance existing on the inside of a tissue.

### Brief Description of Drawings

Fig. 1 is a schematic explanatory view illustrating the structure of the surgical microscope system in accordance with an embodiment of the present invention;
Fig. 2 is a chart for explaining a method for producing output data by the surgical microscope system;
Fig. 3 is a chart illustrating results of capturing images of a subject while changing wavelengths used for first and second image data and generating third image data according to thus captured images;
Fig. 4 is a chart illustrating results of capturing images of a subject while changing wavelengths used for the first and second image data and generating the third image data according to thus captured images;
Fig. 5 is a chart illustrating wavelengths of light selected as wavelengths corresponding to RGB and pseudo-RGB images obtained by combining these wavelengths of light; and
Fig. 6 is a schematic explanatory view illustrating the structure of the surgical microscope system in accordance with a modified example.

### Description of Embodiments

In the following, modes for carrying out the present invention will be explained in detail with reference to the drawings. In the explanation of the drawings, the same constituents will be referred to with the same signs while omitting their overlapping descriptions.

### Surgical Microscope System

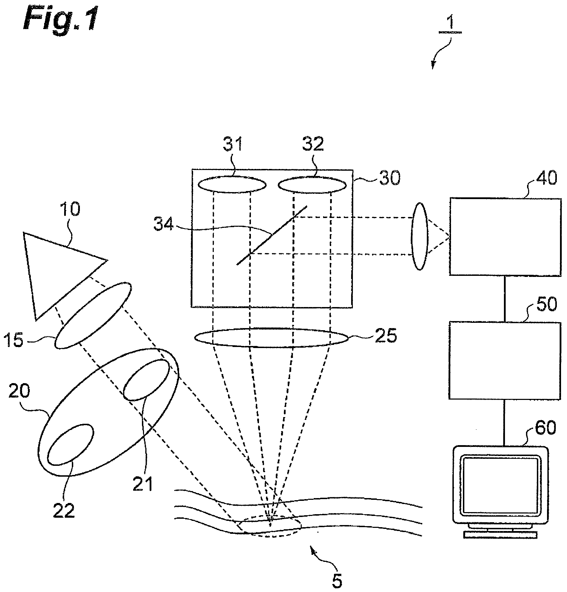

Fig. 1 is a schematic explanatory view illustrating the structure of a surgical microscope system 1 in accordance with an embodiment of the present invention. The surgical microscope system 1 includes a light source 10 (near-infrared light source), a filter unit 20 (optical filter), an observation unit 30, a camera unit 40 (first and second imaging means), a control unit 50 (arithmetic means), and an output unit 60 (display means). This surgical microscope system 1 is a system for non-invasively observing a subject to be observed which is a region hard to observe from the outside. An example of a subject 5 is an inner wall of a blood vessel.

The light source 10 is a light source which emits illumination light at capture wavelengths including at least two wavelength regions within a wavelength range of near-infrared light having a wavelength of 800 nm to 2500 nm; for example, an LD (Laser Diode) light source or SC (Supercontinuum) light source is favorably used therefor. The light source 10 may also be used for capturing shape image data of the subject 5.

The light emitted from the light source 10 is collimated by a collimator lens 15 and then is made incident on the filter unit 20.

The filter unit 20 is arranged on an optical path of the light from the light source 10, inputs the light outputted from the light source 10, transmits therethrough only a specific wavelength of the light according to an instruction from the controller 50, and outputs it to the subject 5. A diffraction grating, a wavelength-variable filter, or the like is used for the filter unit 20. Fig. 1 illustrates a filter wheel including a plurality of filters 21, 22 as an example of the filter unit 20. For the incident light from the light source 10, positions of the filters 21, 22 are changed according to the instruction from the control unit 50, so as to take out the specific wavelength of light and output it to a part to be observed in the subject 5.

The light diffusively reflected at the part to be observed in the subject 5 is turned into parallel light by an objective lens 25 and then is inputted in the observation unit 30. A reflection mirror 34 within the observation unit 30 outputs a part of this light to the camera unit 40.

In the observation unit 30, eyepieces 31, 32 for a user of the surgical microscope system 1 to observe the subject 5 are provided on an optical path of the light turned into the parallel light by the objective lens 25. The user can observe a magnified image of the subject 5 by looking into the eyepieces 31, 32 with the right and left eyes.

The camera unit 40 is means that inputs the light taken out by the reflection mirror 34 and acquiring images concerning the subject 5. Specifically, it has a function as first imaging means that captures an image indicating an intensity distribution of radiation (radiated light) from the subject upon irradiation with the near-infrared light from the light source 10 and outputting image data and a function as second imaging means that caputures an image indicating a surface shape of the subject 5 and outputting shape image data. As the means that acquires the light from the subject 5, a light-receiving element such as a photodiode which converts light into a current and outputs the current is used, for example. The above-mentioned image data acquired by the camera unit 40 is sent to the control unit 50.

The control unit 50 has a function as arithmetic means that produces output data to be outputted in the output unit 60 from the image data concerning the light received in the camera unit 40. A method for producing the output data in the control unit 50 will be explained later. The output data is sent from the control unit 50 to the output unit 60.

The output unit 60 has a function to output the output data produced in the control unit 50. The output unit 60 is constituted by a monitor, for example.

Examples of objects to be observed by the surgical microscope system 1 include plaques adhering to inner walls of blood vessels, thrombosis, and hematoma. Typical examples of the plaques in the inner walls of blood vessels include lipid cores constituted by cholesterol. Plaques and the like adhering to the inner walls of blood vessels are known to narrow and block the blood vessels and cause cerebral infarction, cerebral ischemia, and the like. Therefore, the narrowing or blocking of blood vessels, if any, must be treated with a method of removing plaques from the inner walls of the blood vessels, a method of expanding the blood vessels, and the like. Hence, the surgical microscope system 1 in accordance with this embodiment is mainly aimed at detecting the position of a lipid in the inner wall of a blood vessel as a target substance, thereby non-invasively sensing the existence of a plaque from the outside of the blood vessel.

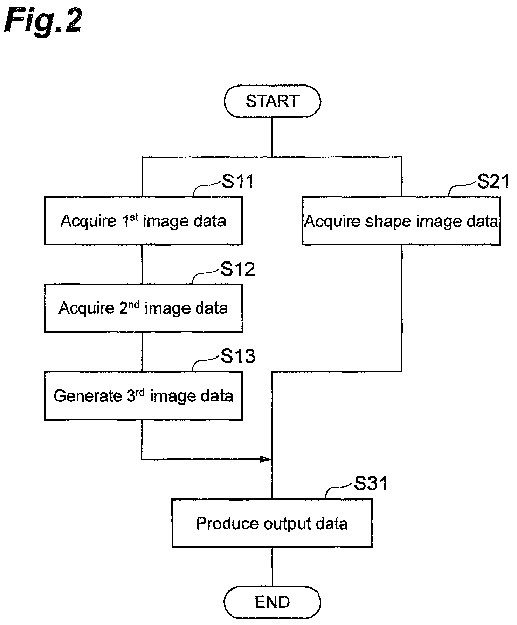

Processing performed in the surgical microscope system 1 for the above-mentioned aim will be explained with reference to Fig. 2. Fig. 2 is a chart for explaining a method for producing output data by the surgical microscope system 1.

The surgical microscope system 1 in accordance with this embodiment performs a series of processing operations concerning the production of image data for specifying the position of the lipid (S11 to S13), acquires shape image data (S21), and then combines them, so as to produce the output data (S31).

First, the production of image data for specifying the position of the lipid will be explained. To begin with, first image data is acquired (S11). The first image data herein is data indicating an intensity distribution of radiation from the subject 5 in a first wavelength region having a width of 50 nm or less containing at least wavelengths within wavelength ranges of 1185 to 1250 nm and 1700 to 1770 nm. The wavelength ranges of 1185 to 1250 nm and 1700 to 1770 nm are wavelength regions having peaks derived from the lipid to be detected in the subject 5. Therefore, employing a wavelength region containing at least the wavelengths within these wavelength ranges as the first wavelength region and acquiring data indicating the intensity distribution of the radiation from the subject 5 in the first wavelength region can detect a region where a large amount of the lipid to be detected exists.

Preferably, the first wavelength region has a bandwidth of 50 nm or less. Such a bandwidth is employed since information concerning an absorption peak not derived from the lipid may be acquired if the bandwidth is greater than 50 nm. In this case, components different from the lipid to be detected may erroneously be detected as the lipid, whereby the accuracy in detecting the lipid may decrease. It is therefore preferable for the bandwidth to be 50 nm or less, so as to acquire information concerning the lipid to be detected with a higher accuracy.

As device structures for acquiring the first image data in the camera unit 40, the light source 10 outputs the near-infrared light including light in the first wavelength region, and the filter 21 of the filter unit 20 transmits therethrough only the light in the first radiation region, whereby the subject 5 is irradiated with the light in the first wavelength region. Then, the light diffusively reflected by the subject 5 is received by the camera unit 40, whereby the first image data can be acquired by the camera unit 40.

Next, second image data is acquired (S 12). The second image data herein is data indicating an intensity distribution of radiation from the subject 5 in a second wavelength region different from the above-mentioned first wavelength region. The second image data is data used for so-called correction employed for eliminating information not derived from the lipid from information contained in the first image data. Therefore, a wavelength region exhibiting less fluctuations with the amount of the lipid as compared with the first wavelength region and indicating a radiation intensity on a par with that derived from a component other than the lipid in the first wavelength region is favorably selected as the second wavelength region. Preferably, such a second wavelength region contains a wavelength region of 1235 to 1300 nm and/or 1600 to 1680 nm. The above-mentioned wavelength range exhibits water absorption on a par with that in the first wavelength region and lipid absorption smaller than that in the first wavelength region and thus can favorably be used in an operation for canceling out the information concerning radiation derived from other components.

As device structures for acquiring the second image data in the camera unit 40, the light source 10 outputs the near-infrared light including light in the second wavelength region, and the filter 22 of the filter unit 20 (changing the filter by rotating the filter wheel) transmits therethrough only the light in the second radiation region, whereby the subject 5 is irradiated with the light in the second wavelength region. Then, the light diffusively reflected by the subject 5 is received by the camera unit 40, whereby the second image data can be acquired by the camera unit 40.

Next, the third image data is generated by using the above-mentioned first and second image data (S13). The third image data is generated by arithmetically operating the radiation intensity of the first image data and the radiation intensity of the second image data for each pixel. Examples of the arithmetic operation include "ratio" (R1/R2, where R1 is the radiation intensity of the first image data, and R2 is the radiation intensity of the second image data), "normalized difference index" ((R1 - R2)/(R1 + R2)), and "difference" (R1 - R2). Performing such an arithmetic operation can produce image data in which peaks derived from the lipid are more emphasized.

Using the "ratio" among them can eliminate the unevenness in the quantity of light among the pixels and the like. Using the "normalized difference index" can express the luminance within the range from -1 to +1 while eliminating the unevenness in the quantity of light and thus can adjust the luminance easily. Using the "difference" can generate the third image data more easily, though with lower accuracy in data, as compared with the "ratio" and "normalized difference index."

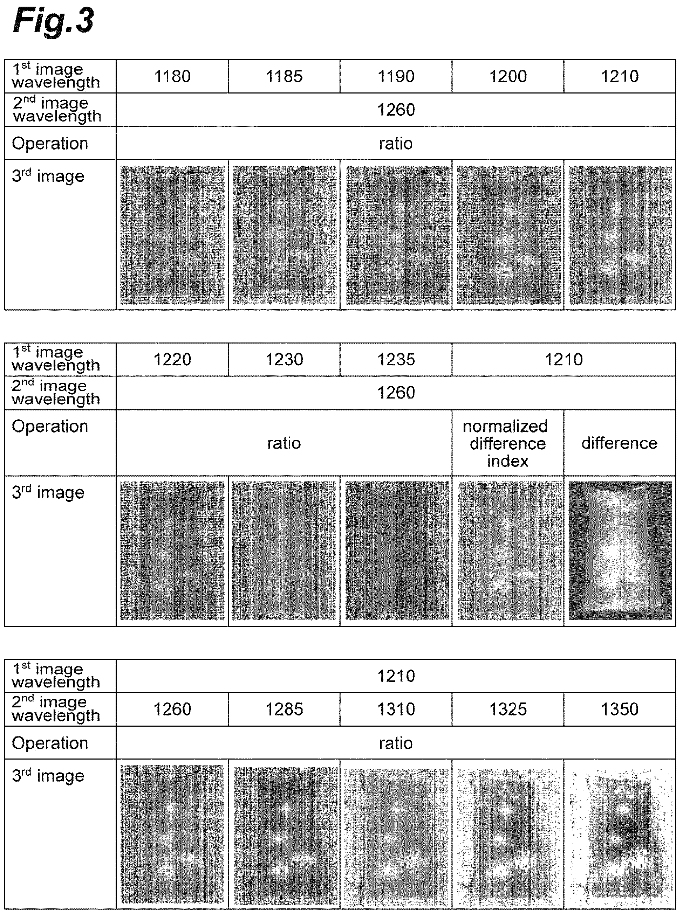

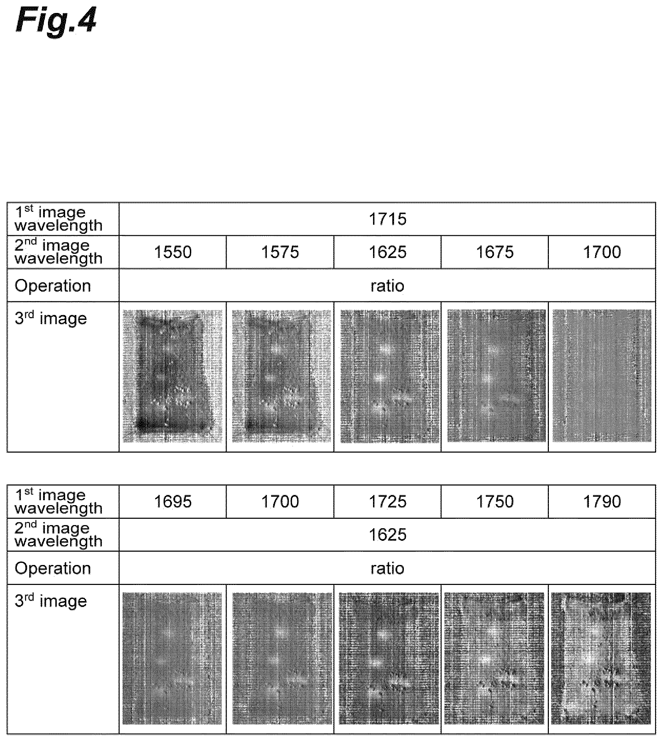

Figs. 3 and 4 illustrate examples in which images of a subject are captured while changing wavelengths used for the first and second image data and the third image data is generated according to the results thereof.

For generating the image data illustrated in Figs. 3 and 4, a part of a region injected with lard between the intima and tunica media of a porcine blood vessel was used as a subject. The first image data was acquired by irradiating the subject with light having a wavelength indicated as the first image wavelength, then the second image data was acquired by irradiating the subject with light having a wavelength indicated as the second image wavelength, and an arithmetic operation was performed for each pixel by the method indicated as the operation, whereby the third image data was obtained. Fig. 3 illustrates the results obtained by selecting one wavelength included in the group consisting of wavelengths of 1180 nm, 1185 nm, 1190 nm, 1200 nm, and 1210 nm as the first image wavelength, selecting one wavelength included in the group consisting of wavelengths of 1260 nm, 1285 nm, 1310 nm, 1325 nm, and 1350 nm as the second image wavelength, and using any of the ratio, normalized difference index, and difference as the arithmetic operation method. Fig. 4 illustrates the results obtained by selecting one wavelength included in the group consisting of wavelengths of 1695 nm, 1700 nm, 1715 nm, 1750 nm, and 1790 nm as the first image wavelength, selecting one wavelength included in the group consisting of wavelengths of 1550 nm, 1575 nm, 1625 nm, 1675 nm, and 1700 nm as the second image wavelength, and using the ratio as the arithmetic operation method.

It is seen from the results illustrated in Figs. 3 and 4 that the third image data capable of specifying the region injected with the lipid (lard) can be obtained by changing the combination of the wavelength used as the first wavelength region and the wavelength used as the second wavelength region.

Returning to Fig. 2, the acquisition of shape image data (S21) will be explained.

The shape image data is image data indicating the shape (form) of the subject 5 in the captured region in the first and second image data. Examples of the image data indicating the shape of the subject 5 include visible light images and pseudo-RGB images. In the case of visible light images, the image data indicating the shape of the subject 5 can be acquired by receiving visible light with the camera unit 40.

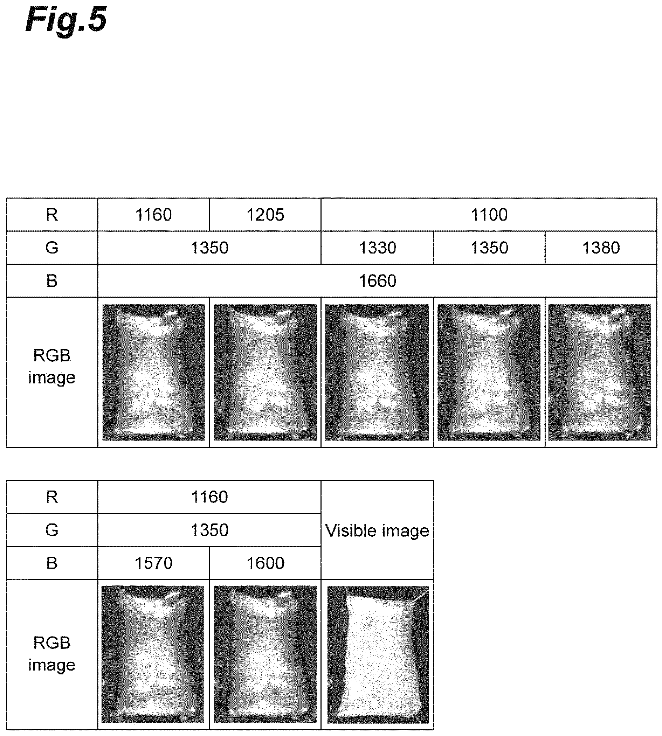

By the pseudo-RGB image is meant an image similar to a visible light image obtained when the intensity distribution per wavelength in each pixel attained upon irradiation of the subject 5 with broadband near-infrared light is caused to correspond to luminances of RGB in a visible region. For example, the received intensity of light having a wavelength within the range of 1100 to 1200 nm is utilized for R, the received intensity of light having a wavelength within the range of 1330 to 1380 nm is utilized for G, and the received intensity of light having a wavelength within the range of 1570 to 1660 nm is utilized for B, whereby the subject 5, which is a biological tissue can be displayed in a tint similar to that of a visible image. In this case, the shape image data can be acquired by emitting the near-infrared light having wavelengths used as RGB from the light source 10 and receiving it with the camera unit 40. Fig. 5 illustrates examples of the above-mentioned pseudo-RGB images. Fig. 5 illustrates wavelengths of light selected as wavelengths corresponding to RGB and the pseudo-RGB images obtained by combinations of these wavelengths of light. It also illustrates a visible image determined from the intensity of visible light. As illustrated in Fig. 5, the shape of the subject 5 can also be grasped in the pseudo-RGB images as in the visible light image.

Once the third image data and shape image data are obtained by the above-mentioned method, the control unit 50 combines them, so as to produce output data (S31). Thus produced output data indicates the region where the lipid exists specified by the third image data as being superposed on the shape image data. In the region where the lipid exists specified by the third image data, an area where the lipid content exceeds a predetermined threshold may be processed alone by coloring and the like. Since the information indicating the region where the lipid exists on the inner wall side is added to the image indicating the shape of the subject 5 in the output data, the information on the inner wall side can be obtained non-invasively even when only the outer shape of the subject 5 is seen while leaving the inner state unknown. When the above-mentioned output data is outputted by the output unit 60, the user can use the information contained in the output data.

When displayed on a monitor or the like in the output unit 60, data concerning pixels in a part of the region indicated to have the lipid that is a target substance may be used for adjusting the luminance of the whole output data. In this case, performing the luminance adjustment in the output data according to the data of the region indicated to have the target substance enables luminance adjustment conforming to the luminance in the surroundings of the target substance, whereby more vivid display can be effected.

As mentioned above, the surgical microscope system 1 in accordance with this embodiment can obtain image data of the third image indicating the position of the target substance from the first image indicating the intensity distribution of the radiation from the subject 5 in the first wavelength region and the second image indicating the intensity distribution of the radiation from the subject in the second wavelength region. The output data obtained by superposing the image data of the third image onto the shape image data further includes information indicating the target substance in addition to the image indicating the surface shape of the subject 5. Therefore, the position of the target substance existing on the inside of a tissue can be grasped non-invasively by referring to the output data. Further, the above-mentioned surgical microscope system 1 can grasp the position of the target substance on the inside of the tissue by capturing the first and second images separately from the shape image data and thus makes it possible to grasp the position of the target substance in a method simpler than conventional methods.

When a wavelength included in the wavelength range of 1235 to 1300 nm and/or 1600 to 1680 nm is selected as the second wavelength region in the above-mentioned surgical microscope system 1, the accuracy in the position of the target substance specified by the third image obtained from the first and second images can further be enhanced.

### Modified Example

A modified example of the surgical microscope system in accordance with an embodiment of the present invention will now be explained. In the following explanation of the modified example, differences from the surgical microscope system 1 illustrated in the above-mentioned embodiment will be explained in particular.

### About the filter unit and light source

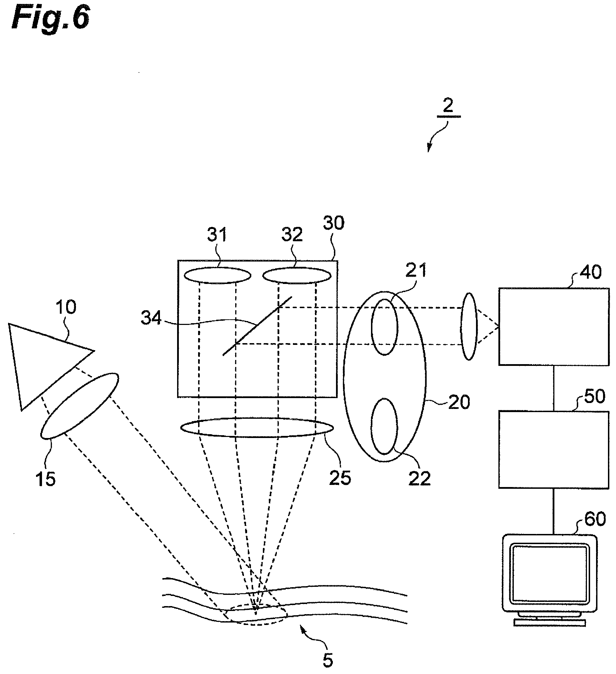

Fig. 6 is a diagram for explaining the structure of a surgical microscope system 2 in accordance with the modified example. The surgical microscope system illustrated in Fig. 6 differs from the surgical microscope system 1 of Fig. 1 in that the position of the filter unit 20 (filter wheel 20) is changed so as to be placed between the observation unit 30 and the camera unit 40.

The surgical microscope system 1 necessitates bright illumination light for observing the subject 5 in general and thus is usually equipped with a heat blocking filter and the like. However, the heat blocking filter and the like cannot always be said to block a specific wavelength of light completely. The method of switching the wavelength of light with the filter unit 20 arranged on the side for irradiating the subject 5 limits the illumination of the surgical illumination light itself, whereby the contrast may decrease. The structure in which the filter unit 20 is provided on the camera unit 40 side, by contrast, can acquire the first and second images without adjusting the wavelength and quantity of light for illuminating the subject 5.

The filter unit 20 may be arranged anywhere on the optical path between the near-infrared light source 10 and the camera unit 40.

The light source 10 itself may be switched instead of limiting the wavelength range of light incident on the camera unit 40 by utilizing the filter. For example, a first light source for emitting light in the first wavelength region and a second light source for emitting light in the second wavelength region may be prepared, so that the first and second images are acquired when the first and second light sources emit light, respectively.

### About the imaging method and arithmetic operation

The imaging method and arithmetic operation method may also be modified in various ways.

For example, when the information indicating the position of the lipid by the above-mentioned surgical microscope system 1 is to be displayed in real time, the above-mentioned embodiment explains a structure acquiring the first image data (S11), acquiring the second image data (S12), and then generating the third image data (S13), but the camera unit 40 may alternately repeat acquiring the first image data (S11) and acquiring the second image data (S12) and, each time one of the first and second image data is acquired, the control unit 50 may generate the third image data (S 13) according to the newest acquired image data and the second-to-newest image data (acquired most recently before acquiring the newest image data).

In this case, since the camera unit 40 alternately acquires the first and second image data, when the newest image data is the first image data, the second-to-newest image data is the second image data, whereby the third image data can be generated by using the latest two sheets of image data. When the operation of generating and outputting the third image data by using the latest two sheets of image data is repeated, shortening the repetition time for acquiring the first and second image data makes it possible to continuously output the third image data indicating the state of the inside of the subject 5, thereby achieving a structure close to real-time display.

For repeating the acquisition of the first image data (S11) and acquisition of the second image data (S12) in the above-mentioned structure, it is preferred for the filter unit 20 to exchange filters in synchronization with timings of acquiring the first image data (S11) and acquiring the second image data (S12), so as to alternately transmit therethrough light in the first wavelength region and light in the second wavelength region.

The system may be configured such that the light source 10 and/or filter unit 20 is driven so as to provide a first time zone for selectively outputting the light in the first wavelength region and a second time zone for selectively outputting the light in the second wavelength region, and the camera unit 40 acquires a plurality of items of first image data in the first time zone and a plurality of items of second image data in the second time zone.

In this case, the control unit 50 may generate the image data of the third image according to data obtained by integrating a plurality of items of the first image data acquired in the first time zone and data obtained by integrating a plurality of items of the second image data acquired in the second time zone.

When the data obtained by integrating a plurality of items of the first image data captured in the first time zone and the data obtained by integrating a plurality of items of the second image data captured in the second time zone are utilized as mentioned above, peaks derived from noise are smoothed in the integrated data, which improves the SN ratio, thereby making it possible to grasp the position of the target substrate with a higher accuracy.

While the above-mentioned embodiment explains a structure in which one camera unit 40 captures the first image, second image, and shape image, imaging means for capturing a near-infrared image (first imaging means) and imaging means for capturing a visible image to become a shape image (second imaging means) may be separated from each other, for example.

### Reference Signs List

1, 2: surgical microscope system; 10: light source; 20: filter unit; 30: observation unit; 40: camera unit; 50: control unit; 60: output unit.

## Claims

1. A surgical microscope system comprising:
a near-infrared light source for emitting illumination light at capture wavelengths including at least two wavelength regions within a wavelength range of 800 nm to 2500 nm;
first imaging means, having a light detection band at the capture wavelengths, the first imaging means configured to capture an image indicating an intensity distribution of radiation from a subject irradiated with the illumination light from the near-infrared light source and to output image data;
second imaging means configured to capture an image indicating a surface shape of the subject at a position provided with the first imaging means and to output shape image data;
arithmetic means configured to produce output data indicating a position of a target substance included in the subject according to the image data output from the first imaging means and the shape image data output from the second imaging means; and
display means configured to display the output data produced by the arithmetic means;
wherein the capture wavelengths include a first wavelength region and second wavelength region, the first wavelength region having a width of 50 nm or less containing at least wavelengths within wavelength ranges of 1185 to 1250 nm and 1700 to 1770 nm and the second wavelength region being different from the first wavelength region;
wherein the first imaging means configured to capture a first image and second image, the first image indicating the intensity distribution of the radiation from the subject in the first wavelength region and the second image indicating the intensity distribution of the radiation from the subject in the second wavelength region and to output respective image data;
wherein the arithmetic means configured to generate image data of a third image indicating the position of the target substance according to the image data of the first image and the image data of the second image and to produce the output data by superposing the image data of the third image onto the shape image data output from the second imaging means.

2. A surgical microscope system according to claim 1, wherein the second wavelength region is included in a wavelength range of 1235 to 1300 nm and/or 1600 to 1680 nm.

3. A surgical microscope system according to claim 1 or 2, wherein the arithmetic means is configured to generate the image data of the third image by calculating a ratio between the intensity of the radiation in the image data of the first image and the intensity of the radiation in the image data of the second image.

4. A surgical microscope system according to any one of claims 1 to 3, wherein the display means is configured to display the output data after adjusting the luminance therein according to data in a part of the region indicated to have the target substance in the third image.

5. A surgical microscope system according to any one of claims 1 to 4, comprising an optical filter, disposed on an optical path from the near-infrared light source to the first imaging means, the optical filter is configured to selectively transmit therethrough light having any of the capture wavelengths including the at least two wavelength regions.

6. A surgical microscope system according to claim 5, wherein the optical filter is configured to alternately selectively transmit therethrough light in the first wavelength region and light in the second wavelength region;
wherein the first imaging means is configured to capture the first and second images during when the optical filter transmits therethrough the light in the first wavelength region and the light in the second wavelength region, respectively, so as to capture the first and second images alternately and output image data; and
wherein the arithmetic means is configured to generate the image data of the third image according to image data acquired as output from the first imaging means and image data acquired most recently before acquiring the former image data.

7. A surgical microscope system according to claim 5, wherein the optical filter is configured to have a first time zone for selectively transmitting therethrough light in the first region and a second time zone for selectively transmitting therethrough light in the second region;
wherein the first imaging means is configured to capture a plurality of the first images in the first time zone and a plurality of the second images in the second time zone; and
wherein the arithmetic means is configured to generate the image data of the third image according to data obtained by integrating image data of the plurality of first images captured in the first time zone and data obtained by integrating image data of the plurality of second images captured in the second time zone.

8. A surgical microscope system according to any one of claims 5 to 7, wherein the optical filter is arranged on an optical path from the subject to the first imaging means

9. A surgical microscope system according to any one of claims 1 to 4, wherein the near-infrared light source includes a first light source for emitting light in the first wavelength region and a second light source for emitting light in the second wavelength region.

10. A surgical microscope system according to claim 9, wherein the near-infrared light source is configured to emit the light in the first wavelength region and the light in the second wavelength region alternately;
wherein the first imaging means is configured to capture the first image during a time when the light in the first wavelength region is emitted and the second images during a time when the light in the second wavelength region is emitted so as to capture the first and second images alternately and output image data; and
wherein the arithmetic means is configured to generate the image data of the third image according to image data acquired as outputted from the first imaging means and image data acquired most recently before acquiring the former image data.

11. A surgical microscope system according to claim 9, wherein the near-infrared light source is configured to have a first time zone for selectively emitting the light in the first region and a second time zone for selectively emitting the light in the second region;
wherein the first imaging means is configured to capture a plurality of first images in the first time zone and a plurality of second images in the second time zone; and
wherein the arithmetic means is configured to generate the image data of the third image according to data obtained by integrating image data of the plurality of first images captured in the first time zone and data obtained by integrating image data of the plurality of second images captured in the second time zone.
